(19) Europäisches Patentamt
European Patent Office
Office européen des brevets

(11) **EP 2 493 325 B1**

(12) **EUROPEAN PATENT SPECIFICATION**

(45) Date of publication and mention
of the grant of the patent:
**02.12.2020 Bulletin 2020/49**

(51) Int Cl.:
*A23C 9/15* *(2006.01)*       *A23J 1/20* *(2006.01)*
*A23L 33/19* *(2016.01)*      *A61P 3/02* *(2006.01)*
*A23C 21/06* *(2006.01)*      *A23C 9/142* *(2006.01)*

(21) Application number: **10781921.1**

(22) Date of filing: **25.10.2010**

(86) International application number:
**PCT/FI2010/050843**

(87) International publication number:
**WO 2011/051557 (05.05.2011 Gazette 2011/18)**

(54) **WHEY PROTEIN PRODUCT AND A METHOD FOR ITS PREPARATION**

MOLKEPROTEINPRODUKT UND VERFAHREN ZU SEINER HERSTELLUNG

PRODUIT À BASE DE PROTÉINES LACTOSÉRIQUES ET SON PROCÉDÉ DE PRÉPARATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.10.2009 FI 20096114
28.10.2009 US 607367**

(43) Date of publication of application:
**05.09.2012 Bulletin 2012/36**

(73) Proprietor: **Valio Ltd
00370 Helsinki (FI)**

(72) Inventors:
• **HARJU, Matti
FI-03100 Nummela (FI)**
• **HEINO, Antti
FI-00390 Helsinki (FI)**
• **TIKANMÄKI, Reetta
FI-00100 Helsinki (FI)**
• **TOSSAVAINEN, Olli
FI-02620 Espoo (FI)**

(74) Representative: **Kolster Oy Ab
(Salmisaarenaukio 1)
P.O. Box 204
00181 Helsinki (FI)**

(56) References cited:
WO-A1-01/03515       WO-A1-96/08155
US-A- 5 085 874       US-A1- 2005 181 095
US-A1- 2007 104 847

• **Belitz & Grosch: "Food Chemistry", 1999,
Springer ISBN: 3-540-64692-2 pages 473-473, *
table 10 ***

## Description

**Field of the invention**

The invention relates to a milk-based product enriched with whey protein and a method for the preparation thereof.

**Background of the invention**

It has been shown that the whey proteins are excellent protein sources, i.a. in nutrition of athletes, in increase and maintenance of muscle mass. Therefore, there are lots of whey protein powders, and beverages produced thereof in the market. In general, as a raw material for said whey protein products, a whey protein concentrate as a powder is used which is prepared by ultrafiltration of cheese, quark, or casein whey and by subsequent drying of the concentrate received from the ultrafiltration. These products have a problem that the taste is foul which results from proteolysis caused by starters such as cheese starters and a rennet, oxidation of residual fat, and other taste flaws. Also, removal of minerals during the production process of the whey products gives rise to a taste which is more watery than that of normal milk. It has been tried to eliminate the problems associated with the taste, whereby the whey products have been flavored up with various food additives, flavoring substances, flavoring preparations and processing aids.

In addition to the taste problems of the current whey protein products, there is a problem that all the whey proteins are not equal in their nutrititive value. For example, nutritive value of glycomacropeptide released from casein into whey during the cheese production is minor than those of $\alpha$-lactalbumin and $\beta$-lactoglobulin. Glycomacropeptide constitutes a significant portion of the total proteins of cheese whey.

Still a further problem arises from the high content of lactose included in the known whey products. As it is commonly known, lactose causes intolerance symptoms for a large amount of adult people in the world.

It is also generally known that thermal treatment of the whey protein based product causes structural faults in the product. These products are typically described as flaky, coarse, lumpy, or sandy.

In view of the above problems, price-quality ratio of the known whey protein products is not attractive. Consequently, the products are not commonly available in large scale but are provided for consumers as specialty products obtainable in restricted facilities, like fitness centers.

Milk-based whey protein products are generally widely known. Also, various membrane techniques and combinations thereof for separating milk components into individual fractions are largely described in the literature. For example, WO 94/13148 discloses a process for producing an undenatured whey protein concentrate by means of microfiltration and ultrafiltration of skim milk. Casein is retained in the microfiltration retentate while $\alpha$-lactalbumin and $\beta$-lactoglobulin penetrate the microfiltration membrane having a pore size of about 0.1 microns quite easily.

WO 96/08155 discloses a separation of casein and whey proteins from a skim milk starting material utilizing microfiltration and ultrafiltration. For example, a milk beverage with a lowered whey protein content can be produced by the process.

WO 00/30461 discloses that microfiltration can be utilized in the preparation of infant formula to make the amino acid composition similar to that of human milk.

WO 03/094623 A1 discloses that several membrane techniques, i.e. ultrafiltration, nanofiltration and reverse osmosis, are utilized to prepare a lactose-free milk beverage.

It is desirable to provide whey protein products that do not possess the drawbacks of the known products but have a pleasant taste and favorable nutritive composition.

**Brief description of the invention**

We have surprisingly found that the problems associated with the known whey products can be avoided by including casein in the milk-based whey protein fraction prepared by membrane techniques and enriched with $\alpha$-lactalbumin and $\beta$-lactoglobulin. It is surprising that even a small amount of casein is sufficient to improve the organoleptic properties of the product, like maintain the taste as smooth and velvety. Surprisingly, also the structure and stability of the whey protein product of the invention is good without any sand, flake, deposition or gel formation etc. Also, the nutritive value of the product is increased.

In an embodiment of the invention, it is possible to prepare a whey protein beverage that looks and tastes like milk but has a composition which is more favorable to athletes and other exercise enthusiasts.

**Brief description of the drawings**

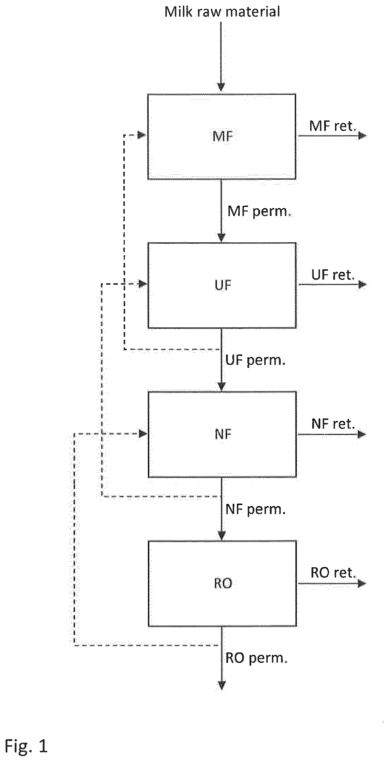

Fig. 1 illustrates an embodiment of the method of the invention for producing a whey protein product.

**Detailed description of the invention**

It is disclosed (not part of the invention) a whey protein product having a ratio of whey protein to casein in the range from about 90:10 to about 50:50 and the total protein content of at least 20% on dry matter basis. An object of the invention is to provide a whey protein product as defined in claim 1. In an embodiment of the invention, the ratio of whey protein to casein ranges from about 80:20 to about 60:40. In a specific embodiment of the invention, said ratio is about 80:20.

In a further embodiment of the disclosure, (not part of the invention), the total protein content of the product ranges from 30% to 60% on dry matter basis. In the invention, the total protein content is 40% to 60% on dry matter basis.

The whey protein product of the invention has good organoleptic properties and, specifically, is free from off-tastes caused by glycomacropeptides and the unpleasant metabolites present in conventional cheese, curd and casein whey. In addition, the whey protein product of the invention possesses favorable nutritive characteristics and favourable effect on health. Also, the stability of the whey protein product of the invention is good where no flakiness, settling, gelling or other phenomena causing undesirable changes in the structure is observed.

In the context of the present invention, the whey protein product means a milk-based protein product containing whey protein and casein. The whey protein product can be prepared from one or more various components obtained from milk raw material by various membrane techniques or a combination thereof. The whey protein product can further comprise minerals of milk origin. The milk raw material can be milk as such or as a concentrate or pretreated as a desired manner. The milk raw material may be supplemented with ingredients generally used in the preparation of milk products, such as fat, protein or sugar fractions. The milk raw material may thus be, for instance, full-fat milk, cream, low-fat milk or skim milk, ultrafiltered milk, diafiltered milk, microfiltered milk, lactose-free or low-lactose milk, protease treated milk, recombined milk from milk powder, organic milk or a combination of these, or a dilution of any of these. Milk can originate from a cow, sheep, goat, camel, horse or any other animal producing milk suitable for nourishment. The milk is preferably low-fat or skim milk. In a more preferred embodiment of the invention, the whey protein product is prepared from skim milk.

The whey protein product of the disclosure (not part of the invention) can be provided as a liquid, like a beverage, a concentrate or a powder. In the invention, the whey protein product is a beverage. The beverage has a typical total protein content of 2.5% to 8% by weight based on the weight of the beverage, preferably 3.5% to 6%. The casein constitutes 5% to 50%, preferably 15% to 25% of the total protein content while the whey protein enriched with $\alpha$-lactalbumin and $\beta$-lactoglobulin constitutes 50% to 95%, preferably 75% to 85%.

It is characteristic of the whey protein product of the invention that it contains no sugar, sweeteners or flavorings, however without limiting to this embodiment. In a specific embodiment of the invention, where the whey protein product is a beverage ready for instant use, no sugar, sweetener or flavoring is included in the beverage.

Like the mineral composition of cow's milk, the mineral composition of the whey protein product of the invention is highly physiological. For example, a whey protein beverage of the invention can typically contain 0.5% to 1.5%, preferably 0.6% to 0.8% of minerals. However, the calcium content of the whey protein product of the invention is lower that that of normal milk. The whey protein product can thus be provided with supplementary calcium and other milk minerals, for example, a nanofiltration permeate received from the process of the invention described below. Supplementary calcium can thus be provided as any calcium source, like milk calcium, calcium gluconate, calcium citrate, calcium lactate etc., or mixtures thereof.

Also fat can be included in the whey protein of the invention. The fat content of the product typically ranges from about 0% up to 3.5%.

In an embodiment of the invention, the whey protein product is low-lactose or lactose-free. The low lactose or lactose-free product can be achieved by membrane techniques used for the preparation of the product. Also, any residual lactose in the whey protein product can be hydrolyzed by means of an enzyme. In the context of the invention, 'low lactose' means a lactose content of less than 1% in the whey protein product. 'Lactose free' means that the lactose content of the whey protein product is 0.5 g/serving (e.g. for liquid milks 0.5 g/244 g, the lactose content being at most 0.21%), however not more than 0.5%. In accordance with the invention, whey protein beverages containing little carbohydrate and having flawless organoleptic characteristics may also be produced.

The whey protein product of the invention can be used as a raw material in the preparation of all kinds of sour milk products and/or acidified fresh products, typically yoghurt, fermented milk, viili and fermented cream, sour cream, quark, butter milk, kefir, dairy shot drinks, and other sour milk products. We surprisingly found that the organoleptic properties of the sour milk products prepared form the whey protein product of the invention are similar to those of conventional sour milk products.

The products of the disclosure (not part of the invention) may be selected from, but are not limited to, the group consisting of food products, animal feed, nutritional products, food supplements, food ingredients, health food and pharmaceutical products. An embodiment of the invention, is the use of the whey protein product in a food or feed or functional food, i.e. food having any health promoting and/or disease preventing and/or alleviating properties. The form of each of the food product, food material, and/or the pharmaceutical products, and the animal feed is not particularly limited.

As stated above, due to its favorable nutritive composition the whey protein product of the invention is suitable for athletes and other exercise enthusiasts as such or as a part of a regular diet. The present invention provides a composition comprising whey protein for supporting and improving healthy eating. The product of the disclosure (not part of the invention) can also be useful especially in connection for alleviation and/or prevention of adult-onset diabetes, metabolic syndrome and sarcopenia.

Another object of the invention is to provide a use of the whey protein product as a food product, animal feed, nutritional product, food supplement, food ingredient and health food. In an embodiment of the invention, the product is provided as a functional food and/or a nutritional product. In an embodiment of the disclosure, (not part of the invention), the product is provided as a pharmaceutical.

The whey protein product is produced from one or more of the fractions obtained by means of membrane techniques. Two or more techniques can be combined, including microfiltration, ultrafiltration, nanofiltration, and reverse osmosis, in an appropriate manner. It is disclosed (not part of the invention), a method for producing a whey protein product which comprises

- subjecting a milk-based raw material to microfiltration to separate an ideal whey as a microfiltration permeate and a casein concentrate as a microfiltration retentate,
- subjecting at least a portion of the microfiltration permeate to ultrafiltration to provide an ultrafiltration permeate and a whey protein concentrate as an ultrafiltration retentate,
- composing a whey protein product from the ultrafiltration retentate and a casein-containing material so as to provide a ratio of whey protein to casein in the range of about 90:10 to about 50:50 and a total protein content of at least 20% on dry matter basis, and if desired, from other ingredients.

An object of the invention is to provide a method for producing a whey protein product as defined in claim 4.

The milk-based raw material is preferably skim milk.

In an embodiment of the invention, the casein-containing material is the microfiltration retentate obtained in the method of the invention. In another embodiment, the casein-containing material is milk. As used herein, the term "milk" means any normal secretion obtained from the mammary glands of mammals, such as cow's, goat's, camel's, horse's or sheep's milk, or any other animal producing milk suitable for nourishment. The milk can be supplemented with ingredients generally used in the preparation of milk products, such as fat, protein or sugar fractions, or the like. The milk thus include, for example, full-fat milk, low-fat milk or skim milk, cream, ultrafiltered milk (UF retentate), diafiltered milk, microfiltered milk (MF permeate), milk recombined from milk powder, organic milk or a combination or dilution of any of these.

In an embodiment, the milk is skim milk. In another embodiment, the milk is low lactose or lactose-free milk.

After composing the whey protein product, it can be heat treated as a manner known per se, if appropriate.

In an embodiment of the invention, at least a portion of the ultrafiltration permeate including majority of the minerals and sugars including lactose can further be subjected to nanofiltration (NF) to separate minerals into a NF permeate and sugars to NF retentate. In another embodiment, at least a portion of the NF permeate can be still further be subjected to reverse osmosis (RO) to concentrate the minerals into a RO retentate. These fractions obtained from said further membrane filtrations can be utilized to compose a whey protein product of the invention. In an embodiment of the invention, a microfiltration retentate, ultrafiltration retentate and nanofiltration permeate are used in the preparation of the whey protein product of the invention. In another embodiment of the invention, a microfiltration retentate, ultrafiltration retentate and reverse osmosis retentate are used in the preparation of the whey protein product of the invention.

In a further embodiment of the invention, microfiltration (MF), ultrafiltration (UF) and/or nanofiltration (NF) are enhanced by diafiltration using water or a suitable fraction obtained from the membrane filtrations. When diafiltration is associated with microfiltration, an UF permeate obtained from the ultrafiltration of the MF permeate is suitably used as diawater. When the UF permeate is further subjected to nanofiltration, a NF permeate is suitably used as diawater in the ultrafiltration. When the NF permeate is still further subjected to reverse osmosis (RO), an RO permeate is suitably used as diawater in the nanofiltration. One or more of said diafiltration steps can be used in the process of the invention.

The method of the invention provides a whey protein product having good organoleptic properties, like taste and mouth-feel, with good stability. It is possible, by means of the method, to prevent the release of glycomacropeptides and metabolites causing unpleasant off-tastes for the whey protein product. It is thus possible to reduce, eliminate or mask the off-tastes of the whey protein product by performing the method of the invention.

The previous studies show that there are differences in nutritive quality of the whey proteins. More particularly, it has been discovered that α-lactalbumin has a more favorable nutritive value than β-lactoglobulin. Based on this knowledge, the composition of the whey protein product of the invention can be adjusted to various uses in an appropriate manner. In the present invention, the adjustment of the whey protein composition is achieved by a heat treatment of milk raw material, or by a selection of a membrane. The process of the invention uses a technique known per se in the heat treatment of milk products. Examples of heat treatments to be used in the process of the invention are pasteurization, high pasteurization, or heating at a temperature lower than the pasteurization temperature for a sufficiently long time. Specifically, UHT treatment (e.g. milk at 138°C, 2 to 4 s), ESL treatment (e.g. milk at 130°C, 1 to 2 s), pasteurization (e.g. milk at 72°C, 15 s), or high pasteurization (95°C, 5 min) can be mentioned. The heat treatment may be either direct (vapour to milk, milk to vapour) or indirect (tube heat exchanger, plate heat exchanger, scraped-surface heat exchanger).

In an embodiment of the invention, milk is subjected to a heat treatment at a temperature range of 65°C to 95°C, for 15 seconds to 10 minutes prior to microfiltration to selectively separate the whey protein ingredients. As a result from the heat treatment, β-lactoglobulin is denatured and associated with casein while α-lactalbumin passes through a membrane. In this way the content of the α-lactalbumin can be increased in the microfiltration permeate.

In an embodiment of the invention, lactose in the whey protein product of the invention in the milk raw material is hydrolyzed into monosaccharides as is well known in the field. This can performed with commercially available lactase enzymes in a manner known per se. In an embodiment of the invention, the lactose hydrolysis is realized after the membrane filtrations on the composed whey protein product. In another embodiment of the invention, the lactose hydrolysis step and microfiltration step are initiated simultaneously with each other. In still another embodiment of the invention, the lactose hydrolysis of the milk raw material is initiated prior to membrane filtration step.

The lactose hydrolysis can continue as long as the lactase enzyme is inactivated, for example by a heat treatment of a whey protein product composed at a later stage of various fractions received in the method of the invention (UF retentate and MF retentate).

The following examples are presented for further illustration of the invention without limiting the invention thereto.

### Example 1

Skim milk (1 000 L) is microfiltered by polymeric filtration membranes (Synder FR) having a pore size of 800 kDa. The concentration factor of 95 is used, including a diafiltration step. The concentration factor is calculated by Equation 1. The amount of microfiltration retentate formed is 190 L having a dry matter content of 20.0%.

$$\text{concentration factor } (-) = \left( \frac{\text{feed} \quad (\text{L})}{\text{retentate} \quad (\text{L})} \right) \quad \text{x} \quad \left( \frac{\text{diafiltration feed} \quad (\text{L})}{\text{diafiltration retentate} \quad (\text{L})} \right) \quad (1)$$

The permeate formed in the microfiltration (1 890 L) is further filtered by polymeric ultrafiltration (UF) membranes (Koch HFK-131) having a pore size of 10 kDa. The permeate obtained from the ultrafiltration is further subjected to nanofiltration (NF) to give a NF retentate and permeate (130 L).

Ultrafiltration is performed by means of diafiltration using 130 L of the NF permeate above as diawater. The total concentration factor of the ultrafiltration is 24 (Equation 1). In the ultrafiltration, 100 L of ultrafiltration retentate and 1 920 L of ultrafiltration permeate are formed, of which 1 080 L is used for the diafiltration of the microfiltration. The remaining ultrafiltration permeate (840 L) is nanofiltered by filtration membranes (Desal 5-DK) having a cut-off value of 200 Da. The concentration factor of the nanofiltration is 4.25 (Equation 1), whereby 197 L of nanofiltration retentate and 644 L of nanofiltration permeate are formed, 130 L of the latter being used as diawater in the diafiltration of the ultrafiltration of the microfiltration permeate, as described above.

The residual nanofiltration permeate not used as diawater in the diafiltration of the ultrafiltration of the microfiltration permeate is used for other purposes or concentrated by reverse osmosis membranes (Koch HR) by using a concentration factor of 10 (Equation 1). The amount of reverse osmosis permeate of the nanofiltration permeate formed is 500 L, of which 44 L is used as diawater in the diafiltration of the nanofiltration. The amount of reverse osmosis retentate of the nanofiltration permeate formed is 55 L.

### Example 2

Skim milk (1 000 L) is subjected to a heat treatment at a temperature range of 65°C to 95°C, for 15 seconds to 10 minutes in a heat treatment apparatus to selectively separate the whey protein ingredients. The heat treatment of the skim milk influences the permeation of whey proteins in the microfiltration so that the microfiltration permeate is enriched with α-lactalbumin that is less thermolabile having denaturation degree of 0 to 26% while β-lactoglobulin is denatured to a degree of 1 to 90%. After the heat treatment of the skim milk, the milk is subjected to the filtration procedures as described in Example 1.

As an example, the proportion of α-lactalbumin of the total amount of α-lactalbumin and β-lactoglobulin (% by weight) in the microfiltration permeate was 38% (heat treatment of 75°C for 30 seconds) to 45% (heat treatment of 90°C for 30 seconds).

**Example 3**

A whey protein product according to the invention was composed from the microfiltration retentate and ultrafiltration retentate of Example 1 as shown in Table 1. The whey protein to casein ratio of the product was 80:20 and the protein content was 58% on the dry matter basis. The product was a low lactose milk drink in which the lactose was hydrolyzed enzymatically after composing.

An educated expert panel evaluated the product organoleptically. The organoleptic properties were 'very good'. No taste flaws or structural faults affecting mouth-feel were observed.

**Table 1**

|  | MF retentate | UF retentate | Product 80:20 low lactose |
|---|---|---|---|
| Portion (%) | 9 | 91 | 100 |
| Protein (%) | 15.3 | 5.8 | 6.6 |
| Whey protein (%) | 0.05 | 5.8 | 5.3 |
| Casein (%) | 15.2 | 0 | 1.4 |
| Lactose (%) | 4.2 | 3.9 | <1 |
| Ash (%) | 3.6 | 0.5 | 0.8 |

**Example 4**

A whey protein product according to the invention was composed from the microfiltration retentate, ultrafiltration retentate and nanofiltration retentate of Example 1, and water as shown in Table 2. The whey protein to casein ratio of the product was 80:20 and the protein content was 43% on the dry matter basis.

An educated expert panel evaluated the product organoleptically. The organoleptic properties were 'very good'. No taste flaws or structural faults affecting mouth-feel were observed.

**Table 2**

|  | MF retentate | UF retentate | NF retentate | Milk mineral powder | Water | Product 80:20 |
|---|---|---|---|---|---|---|
| Portion (%) | 5.2 | 55 | 12 | 0.2 | 27 | 100 |
| Protein (%) | 15.3 | 5.8 | 0 | 0 | 0 | 4.0 |
| Whey protein (%) | 0.05 | 5.8 | 0 | 0 | 0 | 3.2 |
| Casein (%) | 15.2 | 0 | 0 | 0 | 0 | 0.8 |
| Lactose (%) | 4.2 | 3.9 | 17.5 | 45 | 0 | 4.7 |
| Ash (%) | 3.6 | 0.5 | 1.1 | 41 | 0.08 | 0.7 |

**Reference Example 5**

A whey protein product was composed from the microfiltration retentate, ultrafiltration retentate, nanofiltration retentate and nanofiltration permeate of Example 1 as shown in Table 3. The whey protein to casein ratio of the product was 60:40 and the protein content was 38% on dry matter basis.

An educated expert panel evaluated the product organoleptically. The organoleptic properties were 'very good'. No taste flaws or structural faults affecting mouth-feel were observed.

**Table 3**

|  | MF retentate | UF retentate | NF retentate | NF permeate | Product 60:40 |
|---|---|---|---|---|---|
| Portion (%) | 8.7 | 34 | 17 | 40 | 100 |
| Protein (%) | 15.3 | 5.8 | 0 | 0 | 3.3 |
| Whey protein (%) | 0.05 | 5.8 | 0 | 0 | 2.0 |

(continued)

|  | MF retentate | UF retentate | NF retentate | NF permeate | Product 60:40 |
|---|---|---|---|---|---|
| Casein (%) | 15.2 | 0 | 0 | 0 | 1.3 |
| Lactose (%) | 4.2 | 3.9 | 17.5 | 0.08 | 4.7 |
| Ash (%) | 3.6 | 0.5 | 1.1 | 0.2 | 0.8 |

**Example 6**

A whey protein product according to the invention was composed from the microfiltration retentate, ultrafiltration retentate, nanofiltration retentate of Example 1, milk mineral powder and water as shown in Table 4. The whey protein to casein ratio was 50:50 and protein content was 48% on the dry matter basis. The product was a lactose-free milk drink in which the lactose was hydrolyzed enzymatically to a level of less than 0.1% after composing.

An educated expert panel evaluated the product organoleptically. The organoleptic properties were 'very good'. No taste flaws or structural faults affecting mouth-feel were observed.

**Table 4**

|  | MF retentate | UF retentate | NF retentate | Milk mineral powder | Water | Product 50:50 lactose-free |
|---|---|---|---|---|---|---|
| Portion (%) | 11 | 28 | 7.2 | 0.1 | 53 | 100 |
| Protein (%) | 15.3 | 5.8 | 0 | 0 | 0 | 3.3 |
| Whey protein (%) | 0.05 | 5.8 | 0 | 0 | 0 | 1.6 |
| Casein (%) | 15.2 | 0 | 0 | 0 | 0 | 1.6 |
| Lactose (%) | 4.2 | 3.9 | 17.5 | 45 | 0 | <0.1 |
| Ash (%) | 3.6 | 0.5 | 1.1 | 41 | 0.08 | 0.7 |

**Example 7**

A whey protein product according to the invention was composed from the microfiltration retentate, ultrafiltration retentate, nanofiltration retentate and reverse osmosis retentate of Example 1, and water as shown in Table 5. The whey protein to casein ratio of the product was 70:30 and its protein content was 51% on the dry matter basis. The product was a lactose-free milk drink in which the lactose was hydrolyzed enzymatically to a level of less than 0.1% after composing.

An educated expert panel evaluated the product organoleptically. The organoleptic properties were 'very good'. No taste flaws or structural faults affecting mouth-feel were observed.

**Table 5**

|  | MF retentate | UF retentate | NF retentate | RO retentate | Water | Product 70:30 lactose-free |
|---|---|---|---|---|---|---|
| Portion (%) | 7.9 | 48 | 4.4 | 4.4 | 53 | 100 |
| Protein (%) | 15.3 | 5.8 | 0 | 0 | 0 | 4.0 |
| Whey protein (%) | 0.05 | 5.8 | 0 | 0 | 0 | 2.8 |
| Casein (%) | 15.2 | 0 | 0 | 0 | 0 | 1.2 |
| Lactose (%) | 4.2 | 3.9 | 17.5 | 0.8 | 0 | <0.1 |
| Ash (%) | 3.6 | 0.5 | 1.1 | 2.3 | 0.08 | 0.7 |

**Example 8**

A whey protein product according to the invention was composed from milk and the ultrafiltration retentate of Example 1 as shown in Table 6. The whey protein to casein ratio of the product was 70:30 and the protein content was 48% on the dry matter basis.

An educated expert panel evaluated the product organoleptically. The organoleptic properties were 'very good'. No taste flaws or structural faults affecting mouth-feel were observed.

**Table 6**

|  | Milk | UF retentate | Product 70:30 |
|---|---|---|---|
| Portion (%) | 50 | 50 | |
| Protein (%) | 3.4 | 5.8 | 4.6 |
| Whey protein (%) | 0.6 | 5.8 | 3.2 |
| Casein (%) | 2.8 | 0 | 1.4 |
| Lactose (%) | 4.7 | 3.9 | 4.3 |
| Ash (%) | 0.8 | 0.5 | 0.6 |

**Claims**

1. Whey protein product having a ratio of whey protein to casein in the range from 80:20 to 50:50, a total protein content of 40% to 60% on dry matter basis and a protein content of 2.5 to 8% by weight based on the weight of the product, wherein the product is a beverage and wherein the product is produced by a method comprising

   - subjecting a milk-based raw material to microfiltration to separate an ideal whey as a microfiltration permeate and a casein concentrate as a microfiltration retentate,
   - subjecting at least a portion of the microfiltration permeate to ultrafiltration to provide an ultrafiltration permeate and a whey protein concentrate as an ultrafiltration retentate,
   - composing a whey protein product from the ultrafiltration retentate and a casein-containing material so as to provide said whey protein product.

2. The whey protein product of claim 1 wherein the ratio of whey protein to casein is in the range from 80:20 to 60:40, preferably about 80:20.

3. The whey protein product of claim 1 or claim 2 wherein the product further comprises supplementary milk minerals.

4. A method for producing a whey protein product which comprises

   - subjecting a milk-based raw material to microfiltration to separate an ideal whey as a microfiltration permeate and a casein concentrate as a microfiltration retentate,
   - subjecting at least a portion of the microfiltration permeate to ultrafiltration to provide an ultrafiltration permeate and a whey protein concentrate as an ultrafiltration retentate,
   - composing a whey protein product from the ultrafiltration retentate and a casein-containing material so as to provide a ratio of whey protein to casein in the ratio of 80:20 to 50:50, a total protein content of 40% to 60% on dry matter basis, and a protein content of 2.5 to 8% by weight based on the weight of the product, wherein the whey protein product is a beverage.

5. The method of claim 4 wherein the casein-containing material is the microfiltration retentate.

6. The method of claim 4 wherein the casein-containing material is milk.

7. The method of any one of claims 4 to 6 wherein the milk-based raw material is skim milk.

8. The method of any one of claims 4 to 7 wherein the ultrafiltration permeate is subjected to nanofiltration to provide a nanofiltration retentate and permeate.

9. The method of claim 8 wherein the nanofiltration permeate is subjected to reverse osmosis to provide a reverse osmosis retentate and permeate.

10. The method of any one of claims 4 to 9 wherein diafiltration is used with microfiltration, ultrafiltration and nanofiltration.

11. The method of any one of claims 4 to 10 wherein the microfiltration retentate, ultrafiltration retentate and nanofiltration permeate are used for composing the whey protein product.

12. The method of any one of claims 4 to 10 wherein the microfiltration retentate, ultrafiltration retentate and reverse osmosis retentate are used for composing the whey protein product.

13. The method of any one of claims 4 to 12 wherein the milk raw material is subjected to a heat treatment at a temperature range of 65°C to 95°C for 15 seconds to 10 minutes prior to microfiltration.

14. The method of any one of claims 4 to 13 wherein the release of glycomacropeptides in the whey protein product is prevented.

15. The method of any one of claims 4 to 14 wherein the generation of unpleasant metabolites in a whey protein product is prevented.

16. The method of any one of claims 4 to 15 wherein the off-taste of the whey protein product is reduced, eliminated or masked.

17. A use of the whey protein product of any one of claims 1 to 3 or prepared by a method of any one of claims 4 to 16 for use as a food product, animal feed, nutritional product, food supplement, food ingredient and health food.

18. The use of claim 17 wherein the whey protein product is a functional food.

19. A use of the whey protein product of any one of claims 1 to 3 or prepared by a method of any one of claims 4 to 16 in the preparation of sour milk products and/or acidified fresh products, like yoghurt, fermented milk, viili, fermented cream, sour cream, quark, butter milk, kefir, and dairy shot drinks.

**Patentansprüche**

1. Molkenproteinprodukt mit einem Verhältnis von Molkenprotein zu Kasein im Bereich von 80:20 bis 50:50, einem Gesamtproteingehalt von 40 % bis 60 % auf Basis der Trockensubstanz und einem Proteingehalt von 2,5 bis 8 Gew.-%, bezogen auf das Gewicht des Produktes, wobei das Produkt ein Getränk ist und wobei das Produkt mit einem Verfahren hergestellt wird, das umfasst:

   - Unterziehen eines Rohstoffs auf Milchbasis einer Mikrofiltration, um eine ideale Molke als Mikrofiltrationspermeat und ein Kaseinkonzentrat als Mikrofiltrationsretentat zu trennen,
   - Unterziehen zumindest eines Anteils des Mikrofiltrationspermeats einer Ultrafiltration, um ein Ultrafiltrationspermeat und ein Molkenproteinkonzentrat als Ultrafiltrationsretentat bereitzustellen,
   - Zusammensetzen eines Molkenproteinproduktes aus dem Ultrafiltrationsretentat und einem Kasein enthaltenden Material, um das Molkenproteinprodukt bereitzustellen.

2. Molkenproteinprodukt nach Patentanspruch 1, wobei das Verhältnis von Molkenprotein zu Kasein in dem Bereich von 80:20 bis 60:40, vorzugsweise bei etwa 80:20 liegt.

3. Molkenproteinprodukt nach Patentanspruch 1 oder Patentanspruch 2, wobei das Produkt weiter zusätzliche Milchmineralien umfasst.

4. Verfahren zur Herstellung eines Molkenproteinproduktes, umfassend:

   - Unterziehen eines Rohstoffs auf Milchbasis einer Mikrofiltration, um eine ideale Molke als Mikrofiltrationspermeat und ein Kaseinkonzentrat als Mikrofiltrationsretentat zu trennen,
   - Unterziehen zumindest eines Anteils des Mikrofiltrationspermeats einer Ultrafiltration, um ein Ultrafiltrationspermeat und ein Molkenproteinkonzentrat als Ultrafiltrationsretentat bereitzustellen,

- Zusammensetzen eines Molkenproteinproduktes aus dem Ultrafiltrationsretentat und einem Kasein enthaltenden Material, um ein Verhältnis von Molkenprotein zu Kasein im Bereich von 80:20 bis 50:50, einen Gesamtproteingehalt von 40 % bis 60 % auf Basis der Trockensubstanz und einen Proteingehalt von 2,5 bis 8 Gew.-%, bezogen auf das Gewicht des Produktes bereitzustellen, wobei das Molkenproteinprodukt ein Getränk ist.

5. Verfahren nach Patentanspruch 4, wobei das Kasein enthaltende Material das Mikrofiltrationsretentat ist.

6. Verfahren nach Patentanspruch 4, wobei das Kasein enthaltende Material Milch ist.

7. Verfahren nach einem der Patentansprüche 4 bis 6, wobei der Rohstoff auf Milchbasis Magermilch ist.

8. Verfahren nach einem der Patentansprüche 4 bis 7, wobei das Ultrafiltrationspermeat einer Nanofiltration unterzogen wird, um ein Nanofiltrationsretentat und -permeat bereitzustellen.

9. Verfahren nach Patentanspruch 8, wobei das Nanofiltrationspermeat einer Umkehrosmose unterzogen wird, um ein Umkehrosmose-Retentat und -Permeat bereitzustellen.

10. Verfahren nach einem der Patentansprüche 4 bis 9, wobei Diafiltration mit Mikrofiltration, Ultrafiltration und Nanofiltration verwendet wird.

11. Verfahren nach einem der Patentansprüche 4 bis 10, wobei das Mikrofiltrationsretentat, das Ultrafiltrationsretentat und das Nanofiltrationspermeat zum Zusammensetzen des Molkenproteinproduktes verwendet werden.

12. Verfahren nach einem der Patentansprüche 4 bis 10, wobei das Mikrofiltrationsretentat, das Ultrafiltrationsretentat und das Umkehrosmose-Retentat zum Zusammensetzen des Molkenproteinproduktes verwendet werden.

13. Verfahren nach einem der Patentansprüche 4 bis 12, wobei der Milchrohstoff einer Wärmebehandlung in einem Temperaturbereich von 65 °C bis 95 °C für 15 Sekunden bis 10 Minuten vor der Mikrofiltration unterzogen wird.

14. Verfahren nach einem der Patentansprüche 4 bis 13, wobei die Freisetzung von Glykomakropeptiden in dem Molkenproteinprodukt verhindert wird.

15. Verfahren nach einem der Patentansprüche 4 bis 14, wobei die Generation von unangenehmen Metaboliten in dem Molkenproteinprodukt verhindert wird.

16. Verfahren nach einem der Patentansprüche 4 bis 15, wobei der Beigeschmack des Molkenproteinproduktes reduziert, eliminiert oder verdeckt wird.

17. Verwendung des Molkenproteinproduktes nach einem der Patentansprüche 1 bis 3 oder durch ein Verfahren nach einem der Patentansprüche 4 bis 16 hergestellt zur Verwendung als Lebensmittel, Futtermittel, Nahrungsprodukt, Ergänzungslebensmittel, Lebensmittelzutat und Reformkost.

18. Verwendung nach Patentanspruch 17, wobei das Molkenproteinprodukt ein funktionelles Nahrungsmittel ist.

19. Verwendung des Molkenproteinprodukt nach einem der Patentansprüche 1 bis 3 oder durch ein Verfahren nach einem der Patentansprüche 4 bis 16 hergestellt in der Herstellung von Sauermilchprodukten und/oder gesäuerten Frischprodukten, wie Joghurt, fermentierte Milch, Viili, fermentierte Sahne, Sauerrahm, Quark, Buttermilch, Kefir und Dairy-Shot-Getränke.

**Revendications**

1. Produit de protéine de lactosérum ayant un rapport de la protéine de lactosérum à la caséine situé dans la plage allant de 80/20 à 50/50, une teneur en protéine totale de 40 % à 60 % sur la base de matière sèche et une teneur en protéine de 2,5 à 8 % en poids sur la base du poids du produit, lequel produit est une boisson, et lequel produit est produit par un procédé comprenant

- la soumission d'une matière première à base de lait à une microfiltration pour séparer un lactosérum idéal sous la forme d'un perméat de microfiltration et un concentré de caséine sous la forme d'un rétentat de microfiltration,
- la soumission d'au moins une partie du perméat de microfiltration à une ultrafiltration pour former un perméat d'ultrafiltration et un concentré de protéine de lactosérum sous la forme d'un rétentat d'ultrafiltration,
- la composition d'un produit de protéine de lactosérum à partir du rétentat d'ultrafiltration et d'un matériau contenant de la caséine de façon à former ledit produit de protéine de lactosérum.

2. Produit de protéine de lactosérum selon la revendication 1, dans lequel le rapport de la protéine de lactosérum à la caséine est situé dans la plage allant de 80/20 à 60/40, de préférence d'environ 80/20.

3. Produit de protéine de lactosérum selon la revendication 1 ou la revendication 2, lequel produit comprend en outre des minéraux du lait supplémentaires.

4. Procédé de production d'un produit de protéine de lactosérum, qui comprend

    - la soumission d'une matière première à base de lait à une microfiltration pour séparer un lactosérum idéal sous la forme d'un perméat de microfiltration et un concentré de caséine sous la forme d'un rétentat de microfiltration,
    - la soumission d'au moins une partie du perméat de microfiltration à une ultrafiltration pour former un perméat d'ultrafiltration et un concentré de protéine de lactosérum sous la forme d'un rétentat d'ultrafiltration,
    - la composition d'un produit de protéine de lactosérum à partir du rétentat d'ultrafiltration et d'un matériau contenant de la caséine de façon que soit atteint un rapport de la protéine de lactosérum à la caséine de 80/20 à 50/50, une teneur en protéine totale de 40 % à 60 % sur la base de matière sèche et une teneur en protéine de 2,5 à 8 % en poids sur la base du poids du produit, lequel produit de protéine de lactosérum est une boisson.

5. Procédé selon la revendication 4, dans lequel le matériau contenant de la caséine est le rétentat de microfiltration.

6. Procédé selon la revendication 4, dans lequel le matériau contenant de la caséine est du lait.

7. Procédé selon l'une quelconque des revendications 4 à 6, dans lequel la matière première à base de lait est du lait écrémé.

8. Procédé selon l'une quelconque des revendications 4 à 7, dans lequel le perméat d'ultrafiltration est soumis à une nanofiltration pour former un rétentat de nanofiltration et un perméat.

9. Procédé selon la revendication 8, dans lequel le perméat de nanofiltration est soumis à une osmose inverse pour former un rétentat et un perméat d'osmose inverse.

10. Procédé selon l'une quelconque des revendications 4 à 9, dans lequel une diafiltration est utilisée avec une microfiltration, une ultrafiltration et une nanofiltration.

11. Procédé selon l'une quelconque des revendications 4 à 10, dans lequel le rétentat de microfiltration, le rétentat d'ultrafiltration et le perméat de nanofiltration sont utilisés pour composer le produit de protéine de lactosérum.

12. Procédé selon l'une quelconque des revendications 4 à 10, dans lequel le rétentat de microfiltration, le rétentat d'ultrafiltration et le rétentat d'osmose inverse sont utilisés pour composer le produit de protéine de lactosérum.

13. Procédé selon l'une quelconque des revendications 4 à 12, dans lequel la matière première à base de lait est soumise à un traitement thermique à une température située dans la plage allant de 65°C à 95°C pendant 15 secondes à 10 minutes avant la microfiltration.

14. Procédé selon l'une quelconque des revendications 4 à 13, dans lequel la libération de glycomacropeptides dans le produit de protéine de lactosérum est empêchée.

15. Procédé selon l'une quelconque des revendications 4 à 14, dans lequel la génération de métabolites déplaisants dans un produit de protéine de lactosérum est empêchée.

16. Procédé selon l'une quelconque des revendications 4 à 15, dans lequel le faux-goût du produit de protéine de lactosérum est réduit, éliminé ou masqué.

17. Utilisation du produit de protéine de lactosérum de l'une quelconque des revendications 1 à 3 ou préparé par le procédé de l'une quelconque des revendications 4 à 16 pour une utilisation en tant que produit alimentaire, aliment pour animaux, produit nutritionnel, complément alimentaire, ingrédient alimentaire et aliment de santé.

18. Utilisation selon la revendication 17, dans laquelle le produit de protéine de lactosérum est un aliment fonctionnel.

19. Utilisation du produit de protéine de lactosérum de l'une quelconque des revendications 1 à 3 ou préparé par le procédé de l'une quelconque des revendications 4 à 16 dans la préparation de produits lait acide et/ou de produits frais acidifiés, comme le yaourt, le lait fermenté, le viili, la crème fermentée, la crème acide, le quark, le babeurre, le kéfir, et les boissons Dairy-shot.

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9413148 A
- WO 9608155 A
- WO 0030461 A
- WO 03094623 A1